# EUROPÄISCHE PATENTANMELDUNG

(11) **EP 2 027 886 A1**
(43) Veröffentlichungstag der Anmeldung: **25.02.2009**
(21) Anmeldenummer: 08014051.0
(22) Anmeldetag: 06.08.2008
(51) Int. Cl.: A61N 1/05

(54) **Herzschrittmacher-Elektrode mit Schraubwendel und Zuleitungswendel**

(30) Priorität: 22.08.2007 DE 102007039554
(71) Anmelder: Osypka, Peter, 79618 Rheinfelden-Herten (DE)
(72) Erfinder: Osypka, Peter, 79618 Rheinfelden-Herten (DE)
(74) Vertreter: Maucher, Wolfgang

(57) **Zusammenfassung**

Eine Herzschrittmacher-Elektrode (1) weist eine in einem Metallgehäuse (7) angeordnete Schraubwendel (3) auf. Damit bei gegenseitiger Berührung zwischen Schraubwendel (3) und Metallgehäuse (7) kein unerwünschter elektrischer Spannungsimpuls auftreten kann, ist eine dauerhafte elektrische Verbindung des Metallgehäuses (7) mit der Schraubwendel (3) vorgesehen. Diese elektrische Verbindung erfolgt über einen zweckmäßigerweise als Zusatzwendel ausgebildeten elektrischen Leiter (8), der direkt oder indirekt mit einem Teil verbunden ist, dass mit dem drehbaren Steckerstift (4) und der damit verbundenen, zu der Schraubwendel (3) führenden Zuleitungswendel (4) über einen Schleifkontakt (12) ständig elektrisch leitend verbunden ist (Fig. 1).

## Beschreibung

Die Erfindung betrifft eine Herzschrittmacher-Elektrode mit einer während ihrer Implantation zurückgezogenen und durch Drehung aus der zurückgezogenen Lage in eine aktive Halteposition zum Eingreifen in das Herzgewebe in axialer Richtung ausfahrbaren Schraubwendel, die über eine elektrisch leitfähige Zuleitungswendel mit einem in einen Herzschrittmacher einführbaren Steckerstift elektrisch leitend verbunden ist, wobei die axial in Gebrauchsstellung bewegbare Schraubwendel an dem distalen Ende der Herzschrittmacher-Elektrode angeordnet und von einer Halterung in einem Gehäuse gehalten ist, das aus Metall besteht und bei noch in zurückgezogener Lage befindlicher Schraubwendel zum Messen der Reizschwelle am Herzen als Kathode geeignet oder ausgebildet ist, wobei eine dauerhafte elektrische Verbindung des Metallgehäuses mit der Schraubwendel der Herzschrittmacher-Elektrode vorgesehen ist.

Eine vergleichbare Herzschrittmacher-Elektrode ist aus der US 6 687 550 B1 bekannt. Die Schraubwendel befindet sich dabei innerhalb einer als Metallgehäuse wirkenden metallischen Hülse, die an ihrer Stirnseite einen umlaufenden Bund hat. Durch eine zusätzliche Druckfeder soll eine dauerhafte elektrische Verbindung des metallischen Gehäuses mit der Schraubwendel aufrecht erhalten werden. Dabei müssen also an dem distalen Ende der Herzschrittmacher-Elektrode zusätzliche Einbauteile vorgesehen werden, die einen entsprechenden Platzbedarf haben, obwohl es wünschenswert ist, dass vor allem das distale Ende einer solchen Elektrode möglichst klein und platzsparend gestaltet ist.

Es besteht deshalb die Aufgabe, eine Herzschrittmacher-Elektrode der eingangs genannten Art zu schaffen, bei welcher Kontakte zwischen der Schraubwendel und dem sie enthaltenden Metallgehäuse keine Störung des Herzschrittmachers bewirken und dennoch das distale Ende möglichst platzsparend ausgeführt ist.

Zur Lösung dieser Aufgabe ist die eingangs definierte Herzschrittmacher-Elektrode dadurch gekennzeichnet, dass das Metallgehäuse und/oder eine Halterung der axial verstellbaren Schraubwendel mit einem elektrischen Leiter verbunden sind, der entlang der Zuführwendel zu einem proximalen hülsenförmigen Teil oder Hülsenteil führt, relativ zu welchem die Zuleitungswendel und/oder ein Fortsetzungsteil der Zuleitungswendel drehbar ist, und dass das feststehende Hülsenteil und das drehbare Fortsetzungsteil und/oder die drehbare Zuleitungswendel selbst über einen Schleifkontakt elektrisch leitend verbunden sind. Dadurch wird erreicht, dass die elektrische Verbindung des Metallgehäuses mit der Schraubwendel auch während deren Verdrehung aufrechterhalten bleibt. Somit besteht auch nicht die Gefahr, dass zusätzliche Spannungs- oder Stromimpulse durch zusätzliche Berührungen der Schraubwendel und des Metallgehäuses miteinander bewirkt werden. Durch die Verlegung der dauerhaften elektrischen Verbindung zum proximalen Ende der Herzschrittmacher-Elektrode kann außerdem das distale Ende so platzsparend und klein wie möglich gestaltet werden.

Eine effektive Konstruktion kann sich ergeben, wenn der Steckerstift oder das mit dem Steckerstift verbundene Fortsetzungsteil mit der Zuleitungswendel verbunden ist und wenn der Schleifkontakt an einem konzentrisch dazu angeordneten, nicht drehbaren Hülsenteil vorgesehen ist. Somit kann eine relative Verdrehung der Zuleitungswendel, wodurch die Schraubwendel verdreht und gleichzeitig axial verstellt wird, relativ zu der feststehenden Hülse erfolgen, wobei gleichzeitig der Schleifkontakt wirksam ist und für die erfindungsgemäße elektrische Verbindung trotz dieser Verdrehbarkeit sorgt.

Das Fortsetzungsteil des Steckerstifts kann rohrförmig oder hülsenförmig ausgebildet sein und der an dem relativ dazu feststehenden Hülsenteil angeordnete Schleifkontakt kann die Außenseite dieses rohrförmigen Fortsetzungsteils beaufschlagen. Es ist also günstig, wenn das feststehende Hülsenteil den Steckerstift beziehungsweise dessen Fortsetzungsteil außenseitig umfasst und der Schleifkontakt in radialer Richtung zwischen diesen beiden Teilen angeordnet ist.

Der von dem Metallgehäuse und/oder der Halterung der axial verstellbaren Schraubelektrode zu dem Hülsenteil führende elektrische Leiter - womit die dauerhafte elektrische Verbindung des Metallgehäuses mit der Schraubwendel über den Schleifkontakt hergestellt wird - kann eine Zusatzwendel sein, die mit dem die Schraubwendel aufnehmenden Metallgehäuse und/oder der Halterung elektrisch leitend verbunden oder verschweißt ist. Eine derartige Zusatzwendel kann die Entfernung zwischen dem distalen Metallgehäuse und dem im proximalen Bereich der Herzschrittmacher-Elektrode angeordneten Schleifkontakt gut überbrücken und aufgrund ihrer Wendelform die Biegsamkeit und Elastizität der Herzschrittmacher-Elektrode unbeeinflusst und ungestört lassen. Vor allem eventuelle Krümmung der Herzschrittmacher-Elektrode können von dieser Zusatzwendel gut mitgemacht werden, ohne eine derartige eventuell erforderliche Krümmung der Herzschrittmacher-Elektrode zu behindern.

Die Zusatzwendel kann an ihrem proximalen Ende mit dem feststehenden Hülsenteil elektrisch verbunden oder verschweißt sein. Somit wird eine gute Verbindung zu dem Schleifkontakt hergestellt.

Zwischen der drehbaren Zuleitungswendel und der feststehenden Zusatzwendel kann eine schlauchartige oder wendelförmige Führung vorgesehen sein. Diese kann vor allem die Zusatzwendel führen und insbesondere von innen her abstützen und stabilisieren, so dass sie ihre Form und Lage auch bei Bewegungen der Herzschrittmacher-Elektrode beibehält. Gegebenenfalls kann dabei diese Führung sogar auch eine Isolationsfunktion haben oder eine - zusätzliche - Isolierung sein.

Eine sichere und gute Halterung des Endes der Zusatzwendel lässt sich erreichen, wenn das die axial ausfahrbare Schraubwendel und deren Halterung enthaltende Metallgehäuse einen zum proximalen Ende der Herzschrittmacher-Elektrode gerichteten stutzenförmigen oder hohlen Vorsprung aufweist, an welchem die Zusatzwendel elektrisch leitend angreift oder angeschweißt ist und zwar zweckmäßigerweise an der Außenseite. Dieser Vorsprung kann hohl sein, damit die Zuleitungswendel in diesen Vorsprung eingreifen und mit der Halterung der Schraubwendel oder dieser Schraubwendel selbst verbunden sein kann. Gleichzeitig ergibt sich so eine gute und stabile Befestigung für die Zusatzwendel.

Der Schleifkontakt kann durch etwa in axialer Richtung verlaufende, einwärts gekrümmte und unter Spannung und Reibung an dem Gegenstück anliegende Stege gebildet sein, die in Umfangsrichtung eines Hüllkreises nebeneinander angeordnet sind und deren Querschnitte einen in Umfangsrichtung unterbrochenen Kreis beziehungsweise ein Vieleck bilden. Da der Schleifkontakt von außen her an dem drehbaren Steckerstift beziehungsweise dessen Fortsetzungsteil angreift, sind derartige einwärts, also radial nach innen gekrümmte oder bogenförmige Stege besonders gut als Reib- oder Schleifkontakt geeignet. Dabei können diese einstückig miteinander verbunden, aber durch axiale oder gegebenenfalls auch etwas schräge Schlitze voneinander getrennt sein.

Das nicht drehbare elektrisch leitende Hülsenteil kann mit einem Griffstück für den Steckerstift verbunden sein und innerhalb des Griffstückes eine Fortsetzung des Steckerstifts konzentrisch umgreifen, der am proximalen Ende axial gegenüber dem Griffstück vorsteht und relativ zu diesem zusammen mit der Zuleitungswendel verdrehbar ist.

Das Griffstück ist dabei zweckmäßigerweise die bei derartigen Steckern für Herzschrittmacher-Elektroden bekannte Tülle, gegenüber welcher der Steckerstift distal vorsteht. Die erwähnte Fortsetzung kann also innerhalb dieser Tülle oder innerhalb dieses Griffstücks und dabei auch radial innerhalb des den Schleifkontakt aufweisenden oder abstützenden feststehenden Hülsenteils angeordnet sein, um im Bereich des distalen Endes der Herzschrittmacher-Elektrode den erfindungsgemäßen Schleifkontakt unterbringen zu können, über welchen die dauerhafte elektrische Verbindung des Metallgehäuses mit der Schraubwendel erfolgt.

Vor allem bei Kombination einzelner oder mehrerer der vorbeschriebenen Merkmale und Maßnahmen ergibt sich eine Herzschrittmacher-Elektrode, bei welcher trotz einer gewissen Beweglichkeit der Schraubwendel gegenüber dem sie enthaltenden Metallgehäuses bei gelegentlichen Berührungen dieser beiden Teile keine elektrischen Spannungsimpulse erzeugt werden, weil die beiden Teile über die Zusatzwendel ständig elektrisch verbunden sind.

Nachstehend ist ein Ausführungsbeispiel der Erfindung anhand der Zeichnung näher beschrieben. Es zeigt in schematisierter Darstellung:
- Fig. 1: einen Längsschnitt des proximalen Endes der erfindungsgemäßen Herzschrittmacher-Elektrode mit einem aus einem Griffstück eines Steckers proximal vorstehenden, innenseitig hohlen Steckerstift, der sich innerhalb des Griffstückes durch ein Fortsetzungsteil in distaler Richtung bis zu einer Zuleitungswendel erstreckt, wobei das Fortsetzungsteil außenseitig von einem Hülsenteil umschlossen ist, das das Fortsetzungsteil mit einem Schleifkontakt beaufschlagt und elektrisch leitend mit einer Zusatzwendel verbunden ist, die zum distalen Ende der Herzschrittmacher-Elektrode führt,
- Fig. 2: das distale Ende der Herzschrittmacher-Elektrode, in welchem die Zusatzwendel mit dem Metallgehäuse elektrisch leitend verbunden ist, welches die Schraubwendel beziehungsweise deren Halterung enthält, sowie
- Fig. 3: in vergrößertem Maßstab die Ausbildung des Schleifkontakts zur elektrischen Verbindung der Zusatzwendel mit dem Steckerstift und damit mit der Zuleitungswendel.

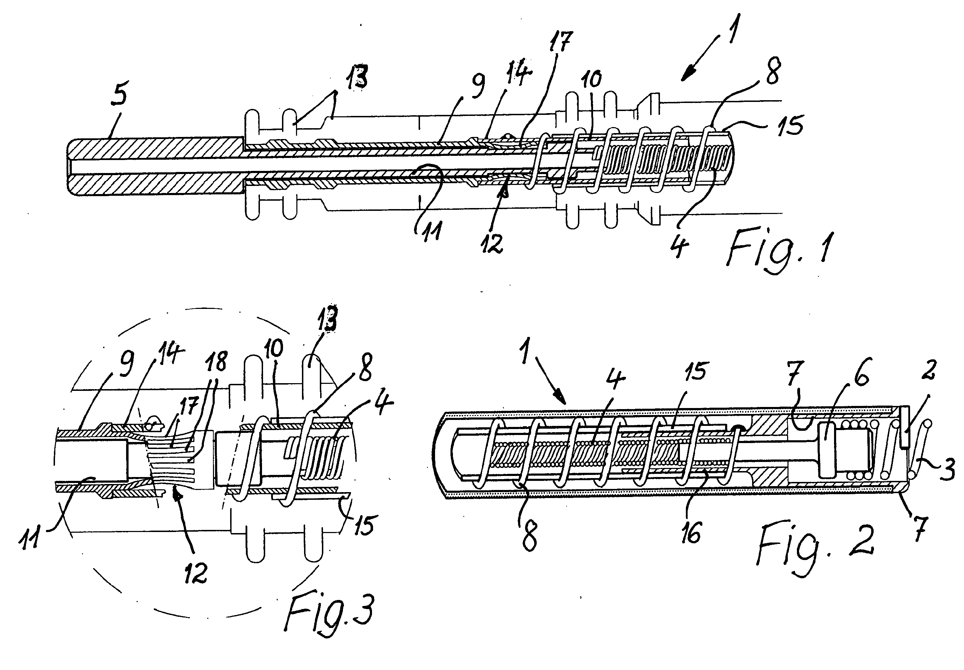

Eine im Ganzen mit 1 bezeichnete Herzschrittmacher-Elektrode, deren wesentliche Teile in den Fig. 1 und 2 dargestellt sind, wobei diese in den Fig. 1 und 2 dargestellten Teile in nicht dargestellter Weise über ein entsprechend langes Zwischenstück verbunden sind, weist eine während ihrer Implantation zurückgezogene und durch Drehung relativ zu einem zwischen ihre Windungen reichenden Eingriffteil 2 aus der zurückgezogenen Lage in eine aktive Halteposition zum Eingreifen in das Herzgewebe in axialer Richtung ausfahrbare Schraubwendel 3 auf, die über eine elektrisch leitfähige Zuleitungswendel 4 mit einem in einen Herzschrittmacher einführbaren, im Ausführungsbeispiel eine Innenlängshöhlung aufweisenden Steckerstift 5 elektrisch leitend verbunden ist.

Die durch die erwähnte Drehung in axialer Richtung in ihre Gebrauchsstellung bewegbare Schraubwendel 3 ist dabei an dem in Fig. 2 dargestellten distalen Ende der Herzschrittmacher-Elektrode 1 angeordnet und von einer Halterung 6 in einem Gehäuse 7 gehalten, das aus Metall besteht und im Folgenden auch "Metallgehäuse 7" genannt wird.

Dieses Metallgehäuse 7 kann bei noch in zurückgezogener Lage befindlicher Schraubwendel 3 zum Messen der Reizschwelle am Herzen als Kathode dienen, wodurch der Operateur auch gleichzeitig erkennen kann, dass die Stirnseite dieses Metallgehäuses 7 in Kontakt mit dem Herzgewebe ist, in welches dann durch Drehung die Schraubwendel 3 eingeschraubt werden soll.

Damit durch Berührungen zwischen der Schraubwendel 3 beziehungsweise ihrer Halterung 6 und dem Metallgehäuse 7 keine elektrische Spannungsimpulse entstehen, ist eine im Folgenden näher beschriebene dauerhafte elektrische Verbindung des Metallgehäuses 7 mit der Schraubwendel 3 vorgesehen. Kommt es zu einer zusätzlichen direkten Berührung zwischen der Schraubwendel 3 und dem Metallgehäuse 7, kann dies nicht zu einem Spannungsimpuls oder Stromimpuls führen, weil beide Teile dauerhaft elektrisch verbunden sind.

Für diese elektrische Verbindung ist ein elektrischer Leiter 8 vorgesehen, der mit dem Metallgehäuse 7 und/oder mit der damit im elektrischen Kontakt befindlichen Halterung 6 der axial verstellbaren Schraubwendel 3 verbunden ist. Dieser von dem Metallgehäuse 7 und/oder der Halterung 6 zu einem noch zu erläuternden Hülsenteil 9 führende elektrische Leiter 8 ist dabei im Ausführungsbeispiel eine Zusatzwendel, die deshalb im Folgenden auch als "Zusatzwendel 8" bezeichnet wird und die mit dem die Schraubwendel 3 aufnehmenden Metallgehäuse 7 und/oder der Halterung6 elektrisch leitend verbunden, im Ausführungsbeispiel in noch zu beschreibender Weise verschweißt ist.

Der wendelförmige Leiter 8 führt dabei entlang der Zuleitungswendel 4 an deren Außenseite zu einem proximalen hülsenförmigen Teil oder Hülsenteil 10, relativ zu welchem die Zuleitungswendel 4 und ein Fortsetzungsteil 11 der Zuleitungswendel 4 drehbar sind, wobei die Hülsenteile 9 und 10 elektrisch leitend verbunden sind und eventuell einstückig sein könnten.

In Fig. 1 und Fig. 3 erkennt man, dass das feststehende Hülsenteil und das drehbare Fortsetzungsteil 11 über einen Schleifkontakt 12 elektrisch leitend verbunden sind, so dass dadurch dann auch die dauerhafte elektrische Verbindung des Metallgehäuses 7 mit der Schraubwendel 3 besteht.

Das Fortsetzungsteil 11 ist dabei mit dem Steckerstift 5 und außerdem, wie schon erwähnt, mit der Zuleitungswendel 4 verbunden und der Schleifkontakt 12 ist an dem konzentrisch dazu angeordneten, nicht drehbaren Hülsenteil 9 vorgesehen, welches im Bereich des Schleifkontakts das hülsenförmige Fortsetzungsteil 11 außenseitig konzentrisch umschließt. Das Fortsetzungsteil 11 des Steckerstifts 5 ist dabei rohrförmig oder hülsenförmig ausgebildet und der an dem relativ dazu feststehenden Hülsenteil 9 angeordnete Schleifkontakt 12 beaufschlagt die Außenseite dieses Fortsetzungsteils 11, wie man es in Fig. 1 erkennt.

Die für die dauerhafte elektrische Verbindung des Metallgehäuses 7 mit der Schraubwendel 3 wesentliche Zusatzwendel 8 ist an ihrem proximalen Ende über das Hülsenteil 10 mit dem feststehenden Hülsenteil 9 elektrisch leitend verbunden. Sie ist dazu mit einem dieses Hülsenteil 9 kontaktierenden das Hülsenteil 10 fortsetzenden Hülsenstück 14 verschweißt, könnte aber auch unmittelbar mit dem Hülsenteil 9 verschweißt sein.

Zwischen der drehbaren Zuleitungswendel 4 und der feststehenden Zusatzwendel 8 erkennt man in den Fig. 1 und 2 jeweils Teile einer sich über die gesamte Herzschrittmacher-Elektrode erstreckenden schlauchartigen oder wendelförmigen Führung 15, durch welche die Zusatzwendel 8 stabilisiert und in ihrer bevorzugten Wendelform gehalten und festgelegt wird, ohne ihre Flexibilität bei Biegungen der Herzschrittmacher-Elektrode zu beeinträchtigen, weil auch diese Führung 15 entsprechend flexibel ist.

In Fig. 2 erkennt man, dass das die axial ausfahrbare Schraubwendel 3 und deren Halterung 6 enthaltende Metallgehäuse 7 einen zum proximalen Ende der Herzschrittmacher-Elektrode 1 gerichteten stutzenförmigen Vorsprung 16 aufweist, an welchem der erfindungsgemäß vorgesehene elektrische Leiter 8, also die Zusatzwendel 8 elektrisch leitend angreift und im Ausführungsbeispiel außenseitig angeschweißt ist.

Bei gemeinsamer Betrachtung der Fig. 1 und 3 wird deutlich, dass der im Ganzen mit 12 bezeichnete Schleifkontakt durch etwa in axialer Richtung verlaufende, einwärts gekrümmte und unter Spannung und Reibung an einem Gegenstück, im Ausführungsbeispiel dem Fortsetzungsteil 11, anliegende Stege 17 gebildet ist, die in Umfangsrichtung eines Hüllkreises nebeneinander angeordnet sind und deren Querschnitte einen in Umfangsrichtung unterbrochenen Kreis beziehungsweise bei mit geradlinigem Querschnitt versehenen Stegen ein entsprechendes Vieleck bilden. Vor allem in Fig. 3 erkennt man zwischen diesen Stegen 17 Schlitze 18, die über den Längenbereich des Schleifkontakts 12 verlaufen und dann enden, um die entsprechende Verformung des Schleifkontakts zu ermöglichen.

Das nicht drehbare, elektrisch leitende Hülsenteil 9 ist im Ausführungsbeispiel mit einem Griffstück 13 für den Steckerstift 5 verbunden, wobei dieses Griffstück 13 von einer üblichen Tülle gebildet ist, die erfasst werden kann, um den Steckerstift 5 in einen Herzschrittmacher einzuführen. Innerhalb dieses Griffstücks 13 umfasst das Hülsenteil 9 das mit dem Steckerstift 3 verbundene Fortsetzungsteil 11 konzentrisch, aber gegebenenfalls mit etwas Spiel oder Abstand, um die Relativverdrehung des Steckerstifts 3 und dieses Fortsetzungsteils 11 zu erlauben, womit die mit diesem Fortsetzungsteil 11 gemäß Fig. 1 verbundene Zuleitungswendel 4 verdreht werden kann, um die Schraubwendel 3 ihrerseits zu verdrehen. In bekannter Weise steht dabei der Steckerstift 3 am proximalen Ende axial gegenüber diesem Griffstück 13 vor, um die beschriebene Relativverdrehung des Steckerstifts 3 zusammen mit der Zuleitungswendel 4 gegenüber diesem Griffstück 13 zu ermöglichen.

Die Herzschrittmacher-Elektrode 1 weist eine in einem Metallgehäuse 7 angeordnete Schraubwendel 3 auf. Damit bei gegenseitiger Berührung zwischen Schraubwendel 3 und Metallgehäuse 7 kein unerwünschter elektrischer Spannungsimpuls auftreten kann, ist eine dauerhafte elektrische Verbindung des Metallgehäuses 7 mit der Schraubwendel 3 vorgesehen. Diese elektrische Verbindung erfolgt über einen zweckmäßigerweise als Zusatzwendel ausgebildeten elektrischen Leiter 8, der direkt oder indirekt mit einem Teil verbunden ist, dass mit dem drehbaren Steckerstift 4 und der damit verbundenen, zu der Schraubwendel 3 führenden Zuleitungswendel 4 über einen Schleifkontakt 12 ständig elektrisch leitend verbunden ist.

## Patentansprüche

1. Herzschrittmacher-Elektrode (1) mit einer während ihrer Implantation zurückgezogenen und durch Drehung aus der zurückgezogenen Lage in eine aktive Halteposition zum Eingreifen in das Herzgewebe in axialer Richtung ausfahrbaren Schraubwendel (3), die über eine elektrisch leitfähige Zuleitungswendel (4) mit einem in einen Herzschrittmacher einführbaren Steckerstift (5) elektrisch leitend verbunden ist, wobei die axial in Gebrauchsstellung bewegbare Schraubwendel (3) in dem distalen Ende der Herzschrittmacher-Elektrode (1) angeordnet und von einer Halterung (6) in einem Gehäuse (7) gehalten ist, das aus Metall besteht und bei noch in zurückgezogener Lage befindlicher Schraubwendel (3) zum Messen der Reizschwelle am Herzen als Kathode geeignet oder ausgebildet ist, wobei eine dauerhafte elektrische Verbindung des Metallgehäuses (7) mit der Schraubwendel (3) der Herzschrittmacher-Elektrode (1) vorgesehen ist, **dadurch gekennzeichnet, dass** das Metallgehäuse (7) und/oder die Halterung (6) der axial verstellbaren Schraubwendel (3) mit einem elektrischen Leiter (8) verbunden sind, der entlang der Zuleitungswendel (4) zu einem proximalen hülsenförmigen Teil oder Hülsenteil (10) führt, relativ zu welchem die Zuleitungswendel (4) und/oder ein Fortsetzungsteil (11) des Steckerstifts (5) und/oder der Zuleitungswendel (4) drehbar sind, und dass das feststehende Hülsenteil (10) und das drehbare Fortsetzungsteil (11) und/oder die drehbare Zuleitungswendel (4) selbst über einen Schleifkontakt (12) elektrisch leitend verbunden sind.

2. Herzschrittmacher-Elektrode nach Anspruch 1, **dadurch gekennzeichnet, dass** der Steckerstift (5) oder das mit dem Steckerstift (5) verbundene Fortsetzungsteil (11) mit der Zuleitungswendel (4) verbunden ist und dass der Schleifkontakt (12) an einem konzentrisch dazu angeordneten, nicht drehbaren Hülsenteil (9) vorgesehen ist.

3. Herzschrittmacher-Elektrode nach einem der Ansprüche 1 bis 2, **dadurch gekennzeichnet, dass** das Fortsetzungsteil (11) des Steckerstifts (5) rohrförmig oder hülsenförmig ausgebildet ist und der an dem relativ dazu feststehenden Hülsenteil (9) angeordnete Schleifkontakt (12) die Außenseite dieses rohrförmigen Fortsetzungsteils (11) beaufschlagt.

4. Herzschrittmacher-Elektrode nach einem der Ansprüche 1 bis 3, **dadurch gekennzeichnet, dass** der von dem Metallgehäuse (7) und/oder der Halterung (6) der axial verstellbaren Schraubelektrode zu dem Hülsenteil (9) führende elektrische Leiter (8) eine Zusatzwendel ist, die mit dem die Schraubwendel (3) aufnehmenden Metallgehäuse (7) und/oder der Halterung (6) elektrisch leitend verbunden oder verschweißt ist.

5. Herzschrittmacher-Elektrode nach einem der Ansprüche 1 bis 4, **dadurch gekennzeichnet, dass** die Zusatzwendel (8) an ihrem proximalen Ende mit dem feststehenden Hülsenteil (9) elektrisch verbunden oder verschweißt ist.

6. Herzschrittmacher-Elektrode nach einem der vorstehenden Ansprüche, **dadurch gekennzeichnet, dass** zwischen der drehbaren Zuleitungswendel (4) und der feststehenden Zusatzwendel (8) eine schlauchartige oder wendelförmige Führung (15) vorgesehen ist.

7. Herzschrittmacher-Elektrode nach einem der Ansprüche 1 bis 6, **dadurch gekennzeichnet, dass** das die axial ausfahrbare Schraubwendel (3) und deren Halterung (6) enthaltende Metallgehäuse (7) einen zum proximalen Ende der Herzschrittmacher-Elektrode (1) gerichteten stutzenförmigen Vorsprung (16) aufweist, an welchem der elektrische Leiter (8), insbesondere die Zusatzwendel, elektrisch leitend angreift oder angeschweißt ist.

8. Herzschrittmacher-Elektrode nach einem der vorstehenden Ansprüche, **dadurch gekennzeichnet, dass** der Schleifkontakt (12) durch etwa in axialer Richtung verlaufende, einwärts gekrümmte und unter Spannung und Reibung an dem Gegenstück anliegende Stege (17) gebildet ist, die in Umfangsrichtung eines Hüllkreises nebeneinander angeordnet sind und deren Querschnitte einen in Umfangsrichtung unterbrochenen Kreis bilden.

9. Herzschrittmacher-Elektrode nach einem der vorstehenden Ansprüche, **dadurch gekennzeichnet, dass** das nicht drehbare, elektrisch leitende Hülsenteil (9) mit einem Griffstück (13) für den Steckerstift (5) verbunden ist und innerhalb des Griffstückes (13) eine Fortsetzungsteil (11) des Steckerstifts (3) konzentrisch umgreift, der am proximalen Ende axial gegenüber dem Griffstück (13) vorsteht und relativ zu diesem zusammen mit der Zuleitungswendel (4) verdrehbar ist.
